# EUROPEAN PATENT SPECIFICATION

(11) **EP 2 952 201 B1**
(45) Date of publication and mention of the grant of the patent: **21.06.2017**
(21) Application number: 14196552.5
(22) Date of filing: 05.12.2014
(51) Int. Cl.: A61K 36/9062, A61K 36/18

(54) **Compositions of Alpinia galanga or Alpinia conchigera with high content of 1'S-1'-acetoxychavicol acetate suitable for pharmaceutical use**
Zusammensetzungen von Alpinia galanga oder Alpinia conchigera mit hohem Gehalt an 1'S-1'-Acetoxychavicolacetat zur medizinischen Verwendung
Compositions d'Alpinia galanga ou Alpinia conchigera à teneur élevée en acétate 1'S-1'-acétoxychavicol approprié pour un traitement pharmaceutique

(30) Priority: 06.06.2014 WO PCT/EP2014/061880
(43) Date of publication of application: 09.12.2015
(73) Proprietor: Nerthus ApS, 4320 Lejre (DK)
(72) Inventor: Jakobsen, Henrik Byrial, 4320 Lejre (DK); Giversen, Ina, 2720 Vanløse (DK)
(74) Representative: Awapatent A/S

(56) References cited:
- JP-A- 2004 189 669
- US-A1- 2002 086 906
- US-A1- 2004 126 441
- US-A1- 2005 238 737
- INDRAYAN A K ET AL: "Nutritive values of some indigenous plant rhizomes resembling Ginger", NATURAL PRODUCT RADIANCE, vol. 8, no. 5, 2009, pages 507-513, XP002727354,
- Alternative Medicine Review Volume, 1 January 2008 (2008-01-01), XP055129347, Retrieved from the Internet: URL:http://www.altmedrev.com/publications/ 13/2/128.pdf [retrieved on 2014-07-16]

## Description

### Field of invention

The present invention relates to a method for preparing a granulate composition of rhizomes from *Alpinia galanga* or *Alpinia conchigera* having a high content of 1'S-1'-acetoxychavicol acetate and low microbiological count, which is suitable for preparing orally ingestible dosage forms such as tablets or capsules. More particularly, the invention relates to a granulate composition of rhizomes of *Alpinia galanga* or *Alpinia conchigera* obtainable by said method containing high levels of 1'S-1'-acetoxychavicol acetate (ACA) which is suitable for subsequent blending with lubricants, binders, disintegrants, and, optionally, additional pharmaceutical active ingredients, such as extracts of *P. granatum,* for tabletization. Also disclosed is the use of such a granulate composition, eg in tablet form, for the treatment of male infertility caused by low sperm count and/or by low sperm motility.

### Background of invention

*Alpinia galanga* (L.) Willd. (or greater galangal) and *Alpinia conchigera* Griff. (or lesser alpinia) belong to the Zingiberaceae (ginger family). The plants are native to Indonesia, Thailand, Malaysia and India. The rhizomes are used as a condiment in some areas. *Alpinia galanga* is traditionally used for the treatment of inflammatory conditions, respiratory infections, cancer, dyspepsia, colic, sea sickness and as a tonic, an aphrodisiac and an abortifacient.

The rhizomes of *A. galanga* and *A. conchigera* comprise several phenylpropanoids with pharmacological activity, including 1'S-1'-acetoxychavicol acetate (ACA), 1'S-1'-acetoxyeugenol acetate (AEA) and 1'S-1'-hydroxychavicol acetate (HCA). The rhizome also contains essential oils with 1,8-cineole being a major constituent. ACA has been reported to have numerous effects as a carcinogenesis inhibitor (Ohnishi et al., 1996), for the treatment of asthma in mice (Seo et al., 2013), as antiplasmid agent (WO07088408 A1), and in the treatment for HIV-1 infection (Ye and Li, 2006). Thus ACA may be an important active component of *A. galanga.*

ACA is a semivolatile phenylpropanoid which is susceptible to evaporation and/or degradation in the course of preparation - especially under typical hydrolytic conditions in water or aqueous ethanol, in particular if raised temperatures are imposed on the extract (Yang and Eilerman, 1999). Under these conditions ACA may be partly or fully converted to 1-hydroxychavicol acetate and/or *p*-acetoxycinnamic alcohol and/or p-coumaryl diacetate (Yang and Eilerman, 1999).

When it comes to male fertility problems, there are two specific problems that tend to occur most often. The first of these is low sperm count. A low sperm count refers to a situation in which a man's semen does not contain a "normal" amount of sperm. A low sperm count is the most common fertility problem for men. After a low sperm count, however, the second most common fertility problem for men is low sperm motility. Low sperm motility refers to a situation in where enough of a man's sperm do not move forward. If the sperm do not move forward, they cannot make the journey from the vagina past the cervix towards the fallopian tubes, where they can fertilize an egg.

There are presently very few medical treatment options for low sperm count and/or low sperm motility, but A. *galanga-*based compositions in general and ACA in particular have for many years been associated with a large variety of pharmacological effects, amongst these recently published results on the effect of a combined pomegranate/galangal-preparation (Punalpin^{®}) on reduced sperm quality in a randomized, placebo-controlled, double-blinded trial. Seventy men with reduced sperm quality were randomized to take tablets containing 1) standardized amounts of rhizome of greater galangal (corresponding to 16 mg 1'S-1'-acetoxychavicol acetate/day) and extract of pomegranate fruit or 2) placebo daily for three months. From baseline to after three months of treatment, the average total number of motile sperm increased with 62% (from 23.4 millions to 37.8 millions) in the pomegranate/galangal-group, while for the placebo group the number of motile sperm increased with 20% (from 19.9 millions to 23.9 millions). The increase in total motile sperm count in the pomegranate/galangal-group was significantly higher than in the placebo group (p= 0.026) (Kaspersen *et al.,* 2013).

A statistical significant improvement of sperm quality, in particular sperm motility (p < 0.01) and sperm count (p < 0.05), has also been reported in healthy mice fed with extract of *A. galanga* (Qureshi et al., 1992). Thus, there is supportive evidence that A. *galanga*-preparations improve sperm motility in mammals.

One possible causative explanation for the beneficial effect of *A.* galanga-preparations on sperm motility is the role of ACA as an antioxidant, more specifically as a superoxide generation inhibitor (Nakamura et al., 1998).

Oxidative stress results from an imbalance between the production of reactive oxygen species (ROS), such as superoxide anion (O₂⁻) and their metabolism, for example, by superoxide dismutase (SOD). Oxidative stress has been shown to exert detrimental effects on sperm quality. ROS-mediated damage to sperm is a significant contributing factor in 30-80% of cases of male infertility. ROS cause infertility by two principal mechanisms. First, ROS damage the sperm membrane, which in turn reduces the sperm's motility and ability to fuse with the oocyte. Secondly, ROS directly damage sperm DNA, compromising the paternal genomic contribution to the embryo (Tremellen 2008).

Another sperm quality-enhancing effect of *A. galanga* relates to testosterone production. A sufficiently high level of testosterone in testis is a prerequisite for male fertility and normal spermatogenesis. The testosterone level in serum increased in rats fed with extract of *A. galanga* (p < 0.05) (Islam et al., 2000). Furthermore, the number of red blood cells increased in mice fed with extract of *A. galanga* (p < 0.05) (Qureshi et al., 1992). The latter effect on red blood cell level may be due to an increase in testosterone production. Testosterone and related androgenic derivatives are known as potent stimulators of red blood cell formation (erythropoiesis).

*Alpinia galanga*-preparations have also been shown to reduce blood glucose in healthy (Akhtar et al., 2002) as well as in diabetic animals (Srividya et al., 2011). Interestingly, diabetes is associated with reduced sperm quality, more specifically increased sperm nuclear and mtDNA damage (Agbaje et al., 2007).

The physiological mechanism behind the effect of *A. galanga* on diabetes has not been determined yet. It may be due to galangin, an antioxidant flavonol present in high concentrations in the rhizomes. The effect of galangin on whole-body insulin resistance and kidney oxidative stress was examined in a fructose-induced rat model of metabolic syndrome (Sivakumar et al., 2010). Galangin dose-dependently normalized blood glucose and insulin levels, and maintained oxidant-antioxidant balance.

Metabolic syndrome is also associated with reduced sperm quality, expressed as reductions in sperm concentration, total sperm count, total motility, sperm vitality, mitochondrial membrane potential, free testosterone and free progesterone, while values for DNA fragmentation increase (Leisegang et al., 2014). Other symptoms of metabolic syndrome are abdominal obesity, high levels of triglycerides in serum, elevated blood pressure, elevated fasting plasma glucose and low high-density lipoprotein (HDL) levels.

Apart from the abovementioned properties of *Alpinia galanga*-preparations regarding improvement of sperm motility, enhancement of testosterone level and reduction of blood glucose level, other studies report on reduction of serum triglycerides (Srividya et al., 2011; lyer et al., 2013), enhancement of HDL levels (lyer et al., 2013) and inhibition of increase in body weight (Kumar et al., 2011). The general anti-hyper-lipidemic activity is possibly caused by the heterocyclic aldehyde, hydroxymethyl-furfural (lyer et al., 2013) and the flavonol galangin (Kumar et al., 2013). All these effects of *A. galanga*-preparations are well-suited to alleviate the symptoms of metabolic syndrome.
Up to date, *A*. *galanga-*based compositions or preparations have been obtained by ethanol or methanol extraction and have shown variable and relatively low contents in ACA, which can be explained either by co-evaporation of ACA with the extraction solvent during workup or by heat-induced decomposition. This has been confirmed by the inventors of the present invention, as the analysis of a number of dry *A. galanga* extracts sold as raw material for food supplements showed very low or no ACA content in the products analyzed. The ACA content in fresh rhizomes is relatively high (up to 11% DW). It is therefore possible that the considerable, or in some cases, total, loss of ACA in the final product, may be caused by either one or more of the methodological steps converting the fresh rhizomes to dry powders suitable for incorporation in tablets, and/or ²⁾ loss during storage prior to or after the preparation of the dry extracts or tablets.
Although ACA is thought to be an important active component of *A. galanga,* it is possible that also other components of *A. galanga* or *A. conchigera* are responsible for the various pharmacological activities referred to above. It is, however, for obvious reasons not feasible to employ fresh rhizomes of *A. galanga* or *A. conchigera* in daily practice. An orally ingestible, dry preparation and/or dosage (eg tablet) form of *A. galanga* and/or *A. conchigera* with a predictable content of phenylpropanoids, notably ACA, and other key components is clearly preferable.

The inventors of the present invention in the international application PCT/EP2014/061880 have described how a dry preparation of rhizomes from *A*. *galanga* or *A. conchigera* can be produced by freeze-drying said rhizomes, followed by pulverizing the dry plant material. The resulting dry preparation contains substantially all the constituent parts of said rhizomes in dry (ie. desiccated) form, including a high content of ACA.

Subsequent work with this dry preparation has however shown that it is not well-suited for preparing orally ingestible dosage forms of *A. galanga* or *A. conchigera* with a high content of ACA, which was the original intention. Firstly, the dry preparation as prepared in PCT/EP2014/061880 has been found to be quite heterogeneous since the fibers of the rhizome have a different density and structure than the remainder components produced by the milling process. It was not possible to incorporate this inhomogeneous mixture of light fibers and heavier components evenly into tablets, i.e., the first tablets produced would have a higher proportion of the heavier fragments compared to those produced at the end of the tablet production process because the smaller and heavier fragments would move to the bottom of the funnel feeding the material into the tablet machine.

Secondly, the large surface area of the pulverized material rendered it susceptible to microbial contamination and at the same time facilitated the evaporation of the volatile compounds originally contained in the rhizomes, including ACA. These factors overall led to a poor stability and shelf life of the bulk material.

Thirdly, as described in PCT/EP2014/061880, in order to prevent microbial contamination of the product, the dry preparation had to undergo a complicated procedure involving heating in airtight bags, and finally, the dry preparation eventually proved very difficult to handle in tablet production due to its poor flowability but especially due to the presence of fine, highly irritant plant fibres which filled the air during handling of the dry preparation, and necessitated the use of ski goggles to prevent eye problems for the operators.

Thus there remains a need for a method for producing a preparation of *A. galanga* and/or *A. conchigera* which not only comprises all the compounds assumed or reported to have pharmacological activity in the original rhizome(s), including a high content of ACA, but also is well suited for pharmaceutical formulation.

### Summary of invention

The present invention is defined by the claims. A method for preparing a substantially anhydrous, granulate composition of *Alpinia galanga* or *Alpinia conchigera* was developed in order to improve the content of phenylpropanoids, ACA in particular, and in order to achieve improved properties for pharmaceutical processing, notably flowability. The method disclosed herein resulted in a granulate composition based on whole rhizomes of *A*. *galanga* having a content of ACA unmatched in literature (8.1% compared with 2.4 % found upon hexane extraction by Yang & Eilerman (1999)) and an even distribution of fibers and other fractions in the bulk granulate. The granulation procedure does not involve any extraction steps, and the resulting granulate composition comprises essentially all the constituent parts originally present in the fresh rhizomes at harvest. Importantly, most microorganisms are removed during the procedure, and the resulting granulate was found to be well suited for tabletization.
The method also allows for the preparation of an oily extract of *Alpinia galanga* or *Alpinia conchigera* having a high content of phenylpropanoids, ACA in particular, which extract is suitable for preparing liquid oral dosage forms.

The disclosure also relates to increasing semen quality in a male subject by administration of said granulate powder or oily extract, eg as tablets or in another orally ingestible form, for example in combination with an extract of *Punica granatum.*

Also disclosed is the use of such a granulate composition or oily extract of *Alpinia galanga* or *Alpinia conchigera,* optionally in combination with an extract of *Punica granatum,* for the treatment of male infertility caused by low sperm count and/or by low sperm motility.

### Definitions

### 1'S-1'-acetoxychavicol acetate (ACA)

1'S-1'-acetoxychavicol acetate (ACA) is a semi-volatile phenylpropanoid. Under typical hydrolytic conditions in water or aqueous ethanol, in particular if raised temperatures are imposed on the extract, ACA may be partly or fully converted to 1'-hydroxychavicol acetate and/or p-acetoxycinnamic alcohol and/or p-coumaryl diacetate.

### Aerobic microorganism

An aerobic organism or aerobe is an organism that can survive and grow in an oxygenated environment.

### Alpinia conchigera

*Alpinia conchigera* Griff. belongs to the Zingiberaceae (ginger family). The plant is native to Thailand, Malaysia and India.

### Alpinia galanga

*Alpinia galanga* (L.) Willd. or greater galangal belongs to the Zingiberaceae (ginger family). The plant is native to Indonesia, Malaysia and India. The plant grows from rhizomes in clumps of stiff stalks up to two meters in height with abundant long leaves and panicles of greenish white flowers.

### Anhydrous/Dry

As understood herein, the terms 'anhydrous' or 'dry' refer to a substance with a water content less than 15%.

### Binder

The term "binder" refers to an excipient, which ensures cohesion within tablets and granules and other formulations. The use of a binder allows formulation with sufficient mechanical strength, and is utilized for converting a powder into **granules** through a process known as Granulation. Granulation is the unit operation by which small powdery particles are agglomerated into larger entities called granules.

### Content Uniformity

As understood herein, the term "content uniformity" or "uniformity of content" is a pharmaceutical analysis technique for the quality control of bulk product, capsules or tablets. Multiple samples, capsules or tablets are selected at random and a suitable analytical method is applied to assay the individual content of the active ingredient in each sample, capsule or tablet.

### Down-sizing

As understood herein, the term "down-sizing" refers to a process wherein a preparation such as a powder undergoes a size reduction. For example, down-sizing of a powder results in a powder wherein the final size of the particles of the powder is reduced. Down-sizing can be performed in conjunction with milling, some millers being equipped with screens which only allow passage of particles smaller than the size of the screen's opening.

### Ellagitannins

Ellagitannins are a diverse class of hydrolyzable tannins, a type of polyphenol formed primarily from the oxidative linkage of galloyl groups in 1,2,3,4,6-pentagalloyl glucose. Ellagitannins have been investigated in cells and animals in laboratories for antioxidant, anti-cancer, antiviral, antimicrobial, and anti-parastite activities, as well as their ability to regulate blood glucose. The pomegranate ellagitannins, which include punicalagin isomers, are ellagitannins found in the fruit, rind (peel), bark or heartwood of pomegranates. Punicalagins are also found to be important for commercial pomegranate juice's antioxidant and health benefits. Examples of ellagitannins found in pomegranates are: punicalins, punicalagin A and B, and punicalin isomers.

### Freeze-drying

Freeze-drying (also known as lyophilization) as understood herein relates to a procedure for drying a solid compound such as rhizomes of *A. galanga.* Freeze-drying procedures as understood herein may comprise the steps of:
i) Freezing the rhizomes to a temperature of about -18 or -20°C;
ii) Applying vacuum until the pressure is stable and in the range of 1,5 to 1,7 mb; the pressure may be maintained stable by supplying e.g. nitrogen;
iii) Increasing the temperature to start the drying process;
iv) Eliminating the vacuum.

Thus, freeze-drying comprises the steps necessary to allow sublimation of the water comprised in the material to be freeze-dried, i.e. the rhizomes. The resulting product contains all the constituent parts of the original rhizome(s) in dry, or desiccated form, ie essentially devoid of water.

### Granulate/Granulation

A granulate or granular material is a conglomeration of discrete solid, macroscopic particles characterized by a loss of energy whenever the particles interact. The constituents that compose a granulate material must be large enough such that they are not subject to thermal motion fluctuations. Thus, the lower size limit for grains in granulate material is about 1 µm. The term 'granulation' refers to the process of converting a powder into a granulate material. A **binder** is typically employed in the granulation process.

### Oily extract

As understood herein, an oily extract of a compound or mixture of compounds refers to a solution of said compound or mixture of compounds in an oil, in particular an edible oil; ie an oil which is considered safe to eat. Typical examples of edible oils are flaxseed oil, olive oil, sunflower oil, corn oil, peanut oil and grape seed oil, all of which typically have a boiling point around 200 °C or higher. Oily extracts as understood herein can be produced by mixing a volume of an edible oil with a solution of said compound or mixture of compounds in an lower boiling organic solvent like ethanol, followed by gentle heating of the mixture under vacuum which cause evaporation of the lower boiling organic solvent, leaving said compound or mixture of compounds in solution in the edible oil.

### Pharmacological activity

Pharmacological activity refers to the effects of a drug on living matter. When a drug is a complex chemical mixture, this activity is exerted by the substance's active ingredient or pharmacophore but can be modified by the other constituents. Activity is generally dosage-dependent.

### Phenylpropanoids

Phenylpropanoids are a diverse family of organic compounds that are synthesized by plants from the amino acid phenylalanine. Their name is derived from the six-carbon, aromatic phenyl group and the three-carbon propene tail of cinnamic acid, which is synthesized from phenylalanine in the first step of phenylpropanoid biosynthesis. Phenylpropanoids are found throughout the plant kingdom, where they serve as essential components of a number of structural polymers, provide protection from ultraviolet light, defend against herbivores and pathogens, and mediate plant-pollinator interactions as floral pigments and scent compounds. Three of the phenylpropanoids found in *Alpinia galanga* are 1'S-1'-acetoxychavicol acetate (ACA), 1'S-1'-acetoxyeugenol acetate (AEA) and 1'-hydroxychavicol acetate (HCA).

### Powder/Pulverisation

A powder is a dry, bulk solid composed of a large number of very fine particles that may flow freely when shaken or tilted. The term 'pulverisation' refers to the process of transforming a solid substance into a powder, e.g. by milling.

### Punicalagins

Punicalagins A and B are a subclass of ellagitannins found to be important for commercial pomegranate juice's antioxidant and health benefits. Punicalagins are also found in other plants of the Combretaceae family: in the leaves of *Terminalia catappa* L., in the fruits of *Terminalia citrina (*Gaertn.*)* Roxb., in the roots of *Terminalia macroptera* Guill. & Perr., in the leaves of *Terminalia myriocarpa* Van Heurck & Müll. Arg., in the leaves of *Terminalis oblongata* F. Muell., in the leaves of *Combretum molle* R. Br. ex G. Don. and in the leaves of *Lumnitzera racemosa* Willd.

### Punicalins

Punicalin is an ellagitannin. The term punicalins as understood herein relates to punicalins A and B as well as punicalin isomers.

### Punicosides

As understood herein, the term punicosides refers to the punicalagins and punicalins, including punicalagin A and B, punicalins A and B and punicalin isomers.

### Rhizome

A rhizome is a modified subterranean stem of a plant that is usually found underground, often sending out roots and shoots from its nodes.

### Semen

Semen, also known as seminal fluid, is an organic fluid that may contain spermatozoa. It is secreted by the gonads (sexual glands) and other sexual organs of male or hermaphroditic animals and can fertilize female ova.

### Semen quality

Semen quality is a measure of the ability of semen to accomplish fertilization. Thus, it is a measure of fertility in a male subject. Semen quality involves both sperm quantity and quality. Decreased semen quality is a major factor of male infertility. Semen quality can be assessed by semen analyses. Examples of parameters measured in a semen analysis are: sperm count, motility, morphology, volume, fructose level and pH.

### Sperm motility

This term refers to the ability of spermatozoa to move forward. In the present context, the term is to be understood as referring also to the motility grade, where the motility of sperm is divided into four different grades:
Grade a: Sperm with progressive motility. These are the strongest and swim fast in a straight line.
Grade b: (non-linear motility): These also move forward but tend to travel in a curved or crooked motion.
Grade c: These have non-progressive motility because they do not move forward despite the fact that they move their tails.
Grade d: These are immotile and fail to move at all.

### Spermatogenesis

Spermatogenesis is the process by which male primordial germ cells called spermatogonia undergo meiosis, and produce a number of cells termed spermatozoa. The initial cells in this pathway are called primary spermatocytes.

### Sperm count, Total motile sperm count (TMSC)

Total motile sperm count (TMSC) or Total motile spermatozoa (TMS) is a combination of sperm count, motility and volume, measuring how many million sperm cells in an entire ejaculate are motile. The TMSC is defined as: ejaculate volume x spermatozoa concentration x percentage of motile spermatozoa.

### Volatility and semi-volatility

Volatility is the tendency of a substance to vaporize. Volatility is directly related to a substance's vapour pressure. At a given temperature, a substance with higher vapour pressure vaporizes more readily than a substance with a lower vapour pressure.

Volatile compounds are compounds that have a high vapour pressure at ordinary, room-temperature conditions. Their high vapour pressure results from a low boiling point, which causes large numbers of molecules to evaporate or sublimate from the liquid or solid form of the compound and enter the surrounding air. A semi-volatile compound is a compound which has a boiling point higher than water and which may vaporize when exposed to temperatures above room temperature.

### Detailed description of the invention

The inventors have now found a solution to overcome the technical problems associated with the dry preparation disclosed in international application PCT/EP2014/061880, which method comprises suspending said dry preparation in an essentially pure organic solvent, such as ethanol, and subjecting the resulting suspension to a wet granulation procedure in the presence of a binder followed by drying and further pharmaceutical processing, eg tabletization. Suspending the dry preparation in ethanol has the effect of preventing microbial growth in the product without resorting to the complicated unit operations described in international application PCT/EP2014/061880. The resulting granulate has a 3-4 times higher density than the starting dry preparation, and thereby a much reduced surface area which is moreover protected by a thin film of binder material. The granulate is a substantially homogenous product with high content uniformity.
Moreover, the granulate displays good flowability measured by its angle of repose, and has proven superior to the original dry preparation for tabletization purposes.

Moreover, during the granulation procedure, which converts the original dry preparation into granules in the presence of a binder, said binder forms a thin coating layer around the granules which may act as a barrier towards evaporation of volatile and semi-volatile compounds present in the granulate, such as ACA. At the same time, the thin coating layer may protect the individual granules from microbial attack and moisture, thereby preventing hydrolysis of labile compounds present in the granulate, such as ACA.
The granulate composition mentioned above is well suited for tabletization purposes, but is not ideal for preparing liquid oral dosage forms. The inventors have now found that by changing a single step of the granulation procedure, an oily extract of *Alpinia galanga* or *Alpinia conchigera* can be prepared which retains a high content of of 1'S-1'-acetoxychavicol acetate (ACA) and has a low microbiological count, which oily extract is well suited for preparing liquid oral dosage forms.

Thus, the invention disclosed herein solves the technical problems associated with the pharmaceutical formulation of the dry preparation as disclosed in application PCT/EP2014/061880.

**In a first aspect** the present disclosure comprising the invention relates to a method for preparing a substantially anhydrous, granulate composition with high content uniformity by wet granulation of a dry preparation of *A. galanga* or *A. conchigera* in the presence of a binder, said granulate composition having a high ACA content and comprising essentially all the constituent parts originally present in the fresh rhizomes at harvest, said method comprising the steps of:
a) providing a dry preparation of *Alpinia galanga* or *Alpinia conchigera* which is milled;
b) suspending said dry preparation in an essentially pure organic solvent,
c) wet granulation of said dry preparation with a binder solution comprising a binder dissolved in an essentially pure organic solvent, said binder solution being essentially devoid of water;
d) removing the organic solvent;
e) provide a final milling;
wherein steps b-d) are performed at a temperature lower than 50°C, such as lower than 40°C, such as lower than 35°C, such as 30°C.

In some embodiments steps b) and c) are combined, such that the dry preparation of *Alpinia galanga* or *Alpinia conchigera* is directly mixed with a binder solution instead of first being suspended in an essentially pure organic solvent.

Evaporation of the non-volatile and semi-volatile components of the *A. galanga* or *A. conchigera* rhizomes such as ACA is avoided in the present granulation procedure because the method does not involve any extraction step. Preferably, the method is performed at low temperatures, thereby reducing the risk of hydrolysis and/or evaporation of ACA and other non-volatile and semi-volatile compounds. Optionally, step d) is carried out in vacuum.

### The dry preparation (step a):

In an embodiment of the invention the dry preparation used in step a) has been prepared from the rhizomes of *Alpinia galanga* or *Alpinia conchigera.*

In a preferred instance the dry preparation of *Alpinia galanga* or *Alpinia conchigera* used in step a) may be provided by the method described in application PCT/EP2014/061880, comprising the following steps:
i) providing non-dried rhizomes of *Alpinia galanga* or *Alpinia conchigera;* and
ii) freeze-drying said rhizomes for a duration such that the water content of said rhizomes is below 15%;
iii) pulverizing said dried rhizomes at a temperature lower than 50°C ;
said dry preparation comprising essentially all the constituent parts originally present in the fresh rhizomes at harvest as a heterogeneous mixture of smaller and larger particles and plant fibres.
Thus, freeze-drying comprises the steps necessary to allow sublimation of the water comprised in the material to be freeze-dried, i.e. the rhizomes.

The resulting freeze-dried rhizomes have a water content less than 15%, such as less than 14%, such as less than 13%, such as less than 12%, such as less than 11%, such as less than 10%, such as less than 9%, such as less than 8%, such as less than 7%, such as less than 6%, such as less than 5%.

Alternatively, it has now been found that the dry preparation of *Alpinia galanga* or *Alpinia conchigera* to be used in step a) may be provided by a method comprising the following steps:
i) providing non-dried rhizomes of *Alpinia galanga* or *Alpinia conchigera;* and
ii) drying said rhizomes in a flow of air for a duration such that the water content of said rhizomes is below 15%;
iii) pulverizing said dried rhizomes at a temperature lower than 50°C ;
said dry preparation comprising essentially all the constituent parts originally present in the fresh rhizomes at harvest as a heterogeneous mixture of smaller and larger particles and plant fibres.

Providing the dry preparation of *Alpinia galanga* or *Alpinia conchigera* by air-flow drying has the advantage of being substantially cheaper than employing the freeze-drying procedure disclosed in PCT/EP2014/061880.

The drying in a flow of air may be carried out at a relative air humidity of the air entering the oven less than 80%, such as 75%, such as 60%, such as 45%, such as 30%, such as 20% or less.

The amount of drying air passing the galanga rhizomes may be adjusted in order to regulate the speed of drying. This is crucial as cells may collapse in the cause of drying thus exposing ACA to free water which may theoretically cause hydrolysis of ACA. The speed of hydrolysis increases with temperature. Thus the optimum drying conditions would be low temperature combined with high air speed and very low air humidity in influx air.

The drying in a flow of air may conveniently be carried out in a vertical drying oven at a drying temperature preferably greater than 25°C, such as 30°C, such as 37°C, such as 40°C, such as 47°C, such as 50°C, such as 52°C, such as 60°C, such as 65°C, such as 70 °C, such as 75°C, such as 80°C, such as 90°C.

In some instances the air flow dried rhizomes have a water content less than 15%, such as less than 14%, such as less than 13%, such as less than 12%, such as less than 11%, such as less than 10%, such as less than 9%, such as less than 8%, such as less than 7%, such as less than 6%, such as less than 5%.
It will be obvious to the skilled man that the temperatures and the durations used for each of the steps involved in the drying procedures may vary depending e.g. on parameters such as the performance of the oven, on the pressure used, on the age of the rhizomes, on the extent of chopping or cutting of the roots.

In a preferred instance the dry preparation of *Alpinia galanga* or *Alpinia conchigera* as discussed in PCT/EP2014/061880 is milled prior to the wet granulation step, using methods known in the art, resulting in a powder.

The milling of the dry preparation is preferably performed at a temperature suitable for preventing hydrolysis and/or evaporation of semi-volatile compounds such as ACA. Thus milling is preferably performed at a temperature lower than 50°C, such as lower than 40°C, such as lower than 35°C, such as lower than 30°C. Without being bound by theory, the inventors hypothesize that high temperatures may accelerate hydrolysis of ACA in the freeze-dried rhizomes, which still contain some water. Thus milling is preferably performed on a miller equipped with a cooling system which can optionally operate in vacuum in order to maintain the temperature within a suitable range despite the milling process being exothermic.

In some instances, the milling step comprises at least one step of down-sizing. The down-sizing may be performed in a miller equipped with a screen, wherein the screen has an opening smaller than 15 mm, such as 12 mm, such as 10 mm, such as 5 mm, such as 4 mm, such as 3 mm, such as 2 mm, such as 1 mm.

In some instances, the at least one step of down-sizing is three steps of down-sizing performed in the following order:
i) down-sizing on a 12 mm screen;
ii) down-sizing on a 2 mm screen;
iii) down-sizing on a 1 mm screen.
Performing multiple steps of down-sizing may facilitate the down-sizing process by first sorting out the bigger particles, whereby further down-sizing of the selected particles is easier.

In some instances, the resulting down-sized powder thus comprises particles having a size smaller than the smallest size of any screen used in the down-sizing process. It will be obvious to the skilled person that the choice of the screen depends on the desired particle size. The step of milling and/or the at least one step of down-sizing preferably result in a substantially homogenous dry preparation, wherein the components of the rhizomes of *A. galanga* or *A. conchigera* are substantially evenly distributed.

### Suspension/wetting of dry preparation (step b)

In order to prevent microbial contamination, the dry preparation obtained as described above and an essentially pure organic solvent such as 99.5% ethanol are combined and stirred prior to subjecting the resulting suspension to the wet granulation procedure described in step c).

In some embodiments this step is omitted, such that the dry preparation of *Alpinia galanga* or *Alpinia conchigera* is directly mixed with a binder solution instead of first being suspended in an essentially pure organic solvent.

### The Wet granulation (step c)

In preparation for this step a binder solution (7.5% polyvinylpyrrolidone (PVP 90) rel. to dry material in 99.5% ethanol) is prepared, typically containing 91-92%% w/w ethanol and 8-9 % w/w PVP90.

The dry preparation or suspension obtained as described above and the PVP-ethanol solution are stirred in a mixer at a temperature not exceeding 27° C. The granulation process typically lasts 1-2 hrs. The ratio between the PVP-ethanol solution and grinded plant material is typically app 1:1 (w/w).

Examples of **suitable binders** include, but are not limited to: saccharides and derivatives thereof: disaccharides, e.g. sucrose or lactose, polysaccharides and derivatives thereof, e.g. starches, cellulose or modified cellulose such as microcrystalline cellulose and cellulose ethers such as hydroxypropyl cellulose (HPC); sugar alcohols and derivatives thereof, e.g. xylitol, sorbitol or maltitol; proteins, e.g. gelatin; semisynthetic polymers such as hydroxypropyl methylcellulose (Hypromellose, or HPMC), synthetic polymers, e.g. polyvinylpyrrolidone (PVP), vinylpyrrolidone-vinyl acetate copolymers (eg Copovidone), polyethylene glycol (PEG). Preferably, the binder is a solution binder. In one embodiment, the binder is PVP, for example PVP90.

In a preferred instance, the binder has good film-forming properties.
**Suitable organic solvents** include solvents which are essentially pure and devoid of water. Without being bound by theory, the inventors believe that it is important that the solvent is devoid of water in order to prevent hydrolysis of ACA and other compounds of *Alpinia galanga* or *Alpinia conchigera.*

In a preferred instance the organic solvent has bactericidal properties.

In some instances, the organic solvent is ethanol or isopropanol. It will be understood that any organic solvent capable of dissolving a binder to obtain a suitable binder solution can be used. In preferred embodiments organic solvents which can be removed by evaporation at a temperature lower than 50°C, such as lower than 40°C, such as lower than 35°C, such as 30°C are employed.
The skilled person will know in which mass ratio the binder should be dissolved in the organic solvent. Thus in some instances, suitable solvent/binder mass ratios are comprised between 80:20 and 98:2, such as 85:15 and 96:4, such as 87:13 and 94:6, such as 89:11 and 92:8, such as 91.5:8.5.

In some instances, the organic solvent is at least 90% pure, such as at least 95% pure, such as at least 96% pure, such as at least 97% pure, such as at least 98% pure, such as at least 99% pure, such as 99.5% pure, such as 100% pure. Thus in some embodiments the organic solvent is ethanol which is at least 90% pure, such as at least 95% pure ethanol, such as at least 96% pure ethanol, such as at least 97% pure ethanol, such as at least 98% pure ethanol, such as at least 99% pure ethanol, such as 99.5% pure ethanol, such as 100% pure ethanol. In other embodiments the organic solvent is isopropanol which is at least 90% pure, such as at least 95% pure isopropanol, such as at least 96% pure isopropanol, such as at least 97% pure isopropanol, such as at least 98% pure isopropanol, such as at least 99% pure isopropanol, such as 99.5% pure isopropanol, such as 100% pure isopropanol.
The skilled person will know in which mass ratio the dry preparation is mixed with the binder solution. In some instances, the binder solution and the dry preparation are contacted at a dry preparation/binder mass ratio comprised between 80:20 and 98:2, such as 85:15 and 96:4, such as 87:13 and 94:6, such as 89:11 and 93:7, such as 92.5:7.5.
Preferably, at least one of steps ii) and iii) is performed at a temperature of 30°C or less. Generally, it is preferable to perform at least one of these steps at a temperature where hydrolysis of ACA and other compounds of *Alpinia galanga* or *Alpinia conchigera* is reduced. Thus in some instances at least steps ii) and iii) are performed at a temperature of 30°C or less. In other embodiments, all of steps i), ii) and iii) are performed at a temperature of 30°C or less.

### Removing the organic solvent (step d)

Removal of the organic solvent takes place directly after the wet granulation process, at a maximum product temperature not more than 30°C, typically under vacuum. Typically the wet granulation mixture is transferred to a fluid bed dryer and the drying process is then carried out with process air (1500 m³/hr) under vacuum conditions. During this process, the product temperature must not exceed 30°C. The whole drying procedure normally lasts 2 - 3 hours.

### Final milling (step e)

The wet granulation process causes the density of the powder to increase 3-4 times *vis-a-vis* the starting dry preparation. After the wet granulation process and removal of the organic solvent, the resulting granules/particles are between 1 - 2 mm. In order to achieve a good tabletizing process resulting in hard tablets with homogenous tablet mass composition, the granulated powder is further milled on a Co-mil using a 1 mm screen. The granulated powder is hereafter suitable for incorporation into tablets.

**In a second aspect,** the present invention also provides a granulate composition of rhizomes from *Alpinia galanga* or *Alpinia conchigera,* said dry preparation comprising:
i) all the constituent parts of *Alpinia galanga* or *Alpinia conchigera* in essentially anhydrous, or desiccated form;
ii) at least 1% 1'S-1'-acetoxychavicol acetate.
The granulate composition of *A. galanga* or *A. conchigera* according to the second aspect of the present invention is obtainable by the method according to the first aspect of the present invention, and is essentially devoid of living microorganisms. In some instances, all the steps of the method for preparing a granulate composition according to the first aspect of the present invention are performed at a temperature of 30°C or less. In some instances, the granulate obtained by the present method is substantially homogenous.

Specific instances of the disclosure have total bacteria counts such that ingestion of the granulate composition according to the second aspect of the present invention is regarded as safe and non-hazardous. For example, *Salmonella* species should be absent from a 25 g sample of the granulate powder, as recommended in general food safety guidelines (Guidelines on the Evaluation of Pathogenic Microorganisms in Food, Ministry for Food, Agriculture and Fishing, Denmark, 1999; Regulation (EC) No 2160/2003 of the European Parliament and of the Council of 17 November 2003 on the control of *Salmonella* and other specified food-borne zoonotic agents). *Escherichia coli* counts should be within the acceptable range of less than 100 per g of preparation. Such preparations are considered essentially devoid of microorganisms.

The compounds which may be comprised in the granulate composition according to the second aspect disclosed herein include, but are not limited to: i) phenylpropanoids, including, but not limited to, 1'S-1'-acetoxychavicol acetate (ACA), 1'S-1'-acetoxyeugenol acetate (AEA) and 1'S-1'-hydroxychavicol acetate (HCA); ii) essential oils, including, but not limited to, 1,8-cineole; iii) minerals, including, but not limited to, magnesium, calcium, potassium and manganese. The dry preparation of *A. galanga* or *A. conchigera* obtainable by the present method may also comprise degradation products of the compounds present in fresh *A. galanga* or *A. conchigera* rhizomes, such as, but not limited to: 1'-hydroxychavicol acetate, p-acetoxycinnamic alcohol, p-coumaryl diacetate. The disclosure further relates to a dry preparation of *A. galanga* or *A. conchigera* comprising one or more of the following: phenylpropanoids, such as 1'S-1'-acetoxychavicol acetate (ACA), 1'S-1'-acetoxyeugenol acetate, 1'S-1'-hydroxychavicol acetate, p-hydroxycinnamaldehyde, p-coumaryl-diacetate, trans-coniferyl-diacetate, trans-p-coumaryl alcohol, trans-p-hydroxycinnamyl acetate, p-acetoxycinnamyl alcohol, p-hydroxybenzaldehyde, chavicol acetate, chavicol, methyl-eugenol, eugenol, eugenol acetate, methyl cinnamate; terpenes and related compounds, including monoterpenes and sesquiterpenes, such as 1,8-cineole, α-pinene, β-pinene, α-terpineol, terpinen-4-ol or 4-terpineol, camphene, camphor, myrcene, (Z)-β-ocimene, limonene, linalool, fenchyl acetate, geranyl acetate, bornyl acetate, citronellyl acetate, 2-acetoxy-1,8-cineole, 3-acetoxy-1,8-cineole, guaiol, β-farnesene, β-bisabolene, (Z,E)-farnesol, β-caryophyllene, α-bergamotene.
The granulate composition according to the second aspect disclosed herein contains at least 1% 1'S-1'-acetoxychavicol acetate, such as at least 1.5% 1'S-1'-acetoxychavicol acetate, such as at least 2% 1'S-1'-acetoxychavicol acetate, such as at least 2.5%, such as at least 3%, such as at least 3.5%, such as at least 4%, such as at least 4.5%, such as at least 5%, such as at least 5.5%, such as at least 6%, such as at least 6.5%, such as at least 7%, such as at least 7.5%, such as at least 8%. Without being bound by theory, the inventors hypothesise that the contents of ACA are indicative of the contents of the components of *A. galanga* or *A. conchigera* which are prone to hydrolysis and/or degradation.
The granulate composition according to the second aspect disclosed herein has a density which is 3-4 times higher than that of the starting dry preparation discussed in PCT/EP2014/061880, and typically a density greater than 12 g/100 mL, such as greater than 15 g/100 mL, such as greater than 20 g/100 mL, such as greater than 22 g/100 mL, such as greater than 25 g/100 mL, such as greater than 26 g/100 mL, such as 27 g/100 mL, such as 28 g/mL, such as 29 g/mL, such as 30 g/mL.
The granulate composition according to the second aspect disclosed herein has a narrower particle size distribution than the starting dry preparation discussed in PCT/EP2014/061880, and displays a better stability of ACA.

In some instances, the granulate composition according to the second aspect disclosed herein has an angle of repose comprised between 30° and 50°, such as between 35° and 45°, such as between 36° and 43°, such as between 37° and 41°, such as between 38° and 40°, such as 39°. The granulate composition of the present invention thus have good flowability characteristics and is well suited for pharmaceutical processing such as eg tabletization.

It is an object of the present disclosure comprising the invention to provide a granulation formulation of *A. galanga* or *A. conchigera* having a high level of 1'S-1'-acetoxychavicol acetate (ACA) and which can be ultimately tabletized by direct compression.

It is a further object of the present disclosure comprising the invention to provide a granulation formulation of *A. galanga* or *A. conchigera* that can be formulated with additional excipients, and, optionally other active ingredients, and compressed into tablets having high hardness, short disintegration time, and fast dissolution rate without being unacceptably friable.

It is a still further object of the disclosure comprising the invention to provide a wet granulation method that produces a granulation formulation of *A. galanga* or *A. conchigera* having a high level of 1'S-1'-acetoxychavicol acetate (ACA) and which is essentially devoid of living microorganisms.

**In a third aspect** the present disclosure relates to a method for preparing a substantially anhydrous, oily extract of *A. galanga* or *A. conchigera,* said oily extract having a high ACA content and comprising essentially all the phenylpropanoids and essential oils originally present in the fresh rhizomes at harvest, including 1'S-1'-acetoxychavicol acetate (ACA), 1'S-1'-acetoxyeugenol acetate (AEA) and 1'S-1'-hydroxychavicol acetate (HCA) and 1,8-cineole, said method comprising the steps of:
a) providing a dry preparation *of Alpinia galanga* or *Alpinia conchigera* which is milled;
b) suspending said dry preparation in an essentially pure organic solvent,
c) stirring the suspension and isolate the extract by filtration of the suspension after a suitable time;
d) mixing the extract with an oil;
e) removing the organic solvent;
wherein steps b-e) are performed at a temperature lower than 50°C, such as lower than 40°C, such as lower than 35°C, such as 30°C.
Evaporation of the non-volatile and semi-volatile components of the *A. galanga* or *A. conchigera* rhizomes such as ACA is substantially avoided in the present procedure because the removal of the relatively volatile organic solvent takes place in the presence of a non-volatile oil, which will dissolve the compounds. Preferably, the method is performed at low temperatures, and optionally, step e) is carried out in vacuum.

In an instance, the oil is selected from non-volatile, edible oils such as flaxseed oil, olive oil, sunflower oil, corn oil, peanut oil and grape seed oil.
**Step a) and Step b)** are performed as described for the first aspect of the invention.

### Isolating extract (step c)

The suspension obtained as described in step b) is stirred in a mixer at a temperature not exceeding 27° C. The extraction process typically lasts 1-2 hrs; the ratio between the essentially pure organic solvent and grinded plant material is normally app 1:1 (w/w). Subsequently the suspension is filtered, and the filter cake rinsed with additional organic solvent. The combined filtrate is used in the next step.

### Mixing the extract with an oil (step d)

The combined filtrate from step c) is mixed with an edible oil such as flaxseed oil, olive oil, sunflower oil, corn oil, peanut oil or grape seed oil, and stirred until the organic solvent - oil mixture is substantially homogeneous. The amount of oil is typically 10-25% (w/w) of the grinded plant material.

### Removing the organic solvent (stepe)

Removal of the organic solvent takes place after the filtrate has been mixed with oil, at a maximum product temperature not more than 30°C, typically under vacuum. Typically the organic solvent - oil mixture is transferred to glass lined reactor or evaporation flask, and the evaporation is conducted with stirring under vacuum conditions. During this process, the product temperature must not exceed 30°C. The progress of the evaporation procedure can be monitored by weighing at regular intervals, and normally lasts 2 - 5 hours depending on scale and equipment.
The oily extract of *A. galanga* or *A. conchigera* obtainable by the method according to the third aspect of the present invention is essentially devoid of living microorganisms. In some embodiments, all the steps of the method for preparing an oily extract according to the third aspect of the present invention are performed at a temperature of 30°C or less.

Specific instances of the invention have total bacteria counts such that ingestion of the oily extract according to the third aspect of the present invention is regarded as safe and non-hazardous. For example, *Salmonella* species should be absent from a 25 g sample of the oily extract, as recommended in general food safety guidelines (Guidelines on the Evaluation of Pathogenic Microorganisms in Food, Ministry for Food, Agriculture and Fishing, Denmark, 1999; Regulation (EC) No 2160/2003 of the European Parliament and of the Council of 17 November 2003 on the control of *Salmonella* and other specified food-borne zoonotic agents). *Escherichia coli* counts should be within the acceptable range of less than 100 per g of preparation. Such preparations are considered essentially devoid of microorganisms.
The compounds which may be comprised in the oily extract according to the third aspect disclosed herein include, but are not limited to: i) phenylpropanoids, including, but not limited to, 1'S-1'-acetoxychavicol acetate (ACA), 1'S-1'-acetoxyeugenol acetate (AEA) and 1'S-1'-hydroxychavicol acetate (HCA); ii) essential oils, including, but not limited to, 1,8-cineole; iii) minerals, including, but not limited to, magnesium, calcium, potassium and manganese. The oily extract of *A. galanga* or *A. conchigera* obtainable by the present method may also comprise degradation products of the compounds present in fresh *A. galanga* or *A. conchigera* rhizomes, such as, but not limited to: 1'-hydroxychavicol acetate, p-acetoxycinnamic alcohol, p-coumaryl diacetate.

The disclosure further relates to an oily extract of *A. galanga* or *A. conchigera* comprising one or more of the following: phenylpropanoids, such as 1'S-1'-acetoxychavicol acetate (ACA), 1'S-1'-acetoxyeugenol acetate, 1'S-1'-hydroxychavicol acetate, p-hydroxycinnamaldehyde, p-coumaryl-diacetate, trans-coniferyl-diacetate, trans-p-coumaryl alcohol, trans-p-hydroxycinnamyl acetate, p-acetoxycinnamyl alcohol, p-hydroxybenzaldehyde, chavicol acetate, chavicol, methyl-eugenol, eugenol, eugenol acetate, methyl cinnamate; terpenes and related compounds, including monoterpenes and sesquiterpenes, such as 1,8-cineole, α-pinene, β-pinene, α-terpineol, terpinen-4-ol or 4-terpineol, camphene, camphor, myrcene, (Z)-β-ocimene, limonene, linalool, fenchyl acetate, geranyl acetate, bornyl acetate, citronellyl acetate, 2-acetoxy-1,8-cineole, 3-acetoxy-1,8-cineole, guaiol, β-farnesene, β-bisabolene, (Z,E)-farnesol, β-caryophyllene, α-bergamotene.
The oily extract according to the third aspect disclosed herein contains at least 1% 1'S-1'-acetoxychavicol acetate, such as at least 1.5% 1'S-1'-acetoxychavicol acetate, such as at least 2% 1'S-1'-acetoxychavicol acetate, such as at least 2.5%, such as at least 3%, such as at least 3.5%, such as at least 4%, such as at least 4.5%, such as at least 5%, such as at least 5.5%, such as at least 6%, such as at least 6.5%, such as at least 7%, such as at least 7.5%, such as at least 8%. Without being bound by theory, the inventors hypothesise that the contents of ACA are indicative of the contents of the components of *A. galanga* or *A. conchigera* which are prone to hydrolysis and/or degradation.
Liquid dosage forms for oral administration of the oily extract of *A. galanga* or *A. conchigera* according to the third aspect of the present invention include solutions, emulsions, suspensions, syrups and elixirs.
For illustrative purposes, a typical liquid dosage form for oral administration may contain from 5 mg 1'S-1'-acetoxychavicol acetate/day, such as between 5 and 50 mg 1'S-1'-acetoxychavicol acetate/day, preferably at least 10 mg 1'S-1'-acetoxychavicol acetate/day contained in an oily extract of *A. galanga* or *A. conchigera* according to the third aspect of the present invention.

### Pharmaceutical compositions

**In a fourth aspect** the present disclosure comprising the invention provides a pharmaceutical composition comprising a therapeutically effective amount of the granulate composition or oily extract of *A. galanga* or *A. conchigera* herein disclosed, and a pharmaceutically acceptable carrier or diluent.

The pharmaceutical compositions may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995.
Pharmaceutical compositions for oral administration include solid dosage forms such as capsules, tablets, dragees, pills, lozenges, powders and granules. Where appropriate, the compositions may be prepared with coatings such as enteric coatings or they may be formulated so as to provide controlled release of the active ingredient such as sustained or prolonged release according to methods well known in the art.
Typical oral dosages of a granulate composition or the oily extract herein disclosed range from at least 100 mg/day, such as at least 125 mg/day, such as at least 250 mg/day, such as between 250 and 3000 mg/day, preferably at least 500 mg/day, such as 225 mg/day. Oral dosages are usually administered in one or more dosages, typically, one to three dosages per day. The exact dosage will depend upon the frequency and mode of administration, the age, weight and general condition of the mammal treated, the nature and severity of the condition treated and any concomitant diseases to be treated and other factors evident to those skilled in the art.
The formulations may also be presented in a unit dosage form by methods known to those skilled in the art. For illustrative purposes, a typical unit dosage form for oral administration may contain from 5 mg 1'S-1'-acetoxychavicol acetate/day, such as between 5 and 50 mg 1'S-1'-acetoxychavicol acetate/day, preferably at least 10 mg 1'S-1'-acetoxychavicol acetate/day contained in a granulate composition or oily extract of *A. galanga* or *A. conchigera.*

Also within the scope of the present invention is a method for preparing an ingestible preparation, such as, but not limited to, a tablet, a pill, a capsule or a powder, from the granulate composition or the oily extract herein disclosed. The ingestible preparation may be formulated as a dietary supplement, a food additive or as a medical food. The methods for formulating the granulate composition may be any method known by the skilled man. The formulation may comprise other ingredients and may comprise coating. It will be understood that the granulate composition may also be suspended in e.g. edible oil prior to formulation.

In some instances, any additional formulation steps are performed at a temperature lower than 50°C, such as lower than 40°C, such as lower than 35°C, such as lower than 30°C.
Formulations of the present invention suitable for oral administration may be presented as discrete units such as capsules or tablets, each containing a predetermined amount of the active ingredient, and optionally a suitable excipient. Furthermore, the orally available formulations may be in the form of a powder or granules, a solution, extract or suspension in an aqueous or non-aqueous liquid, or an oil-in-water or water-in-oil liquid emulsion.

If a solid carrier is used for oral administration, the preparation may be tabletted, placed in a hard gelatin capsule in powder or pellet form or it may be in the form of a troche or lozenge. If a liquid carrier is used, the preparation may be in the form of a syrup, emulsion or soft gelatin capsule, or an aqueous or non-aqueous liquid suspension.
The pharmaceutical composition may be formulated as an ingestible preparation, such as a tablet, a pill, a capsule or a powder, or as a dietary supplement or a food additive or a medical food, or as a suspension in eg an edible oil.

The ingestible preparations (eg tablets) prepared from the granulate according to the second aspect of the present invention may further be coated with a coating agent. Suitable coating agents are known in the art. Water-based coating agents may be employed. Such coating agents do not appear to accelerate hydrolysis of ACA. It will be obvious to the skilled person that other agents such as fillers, anti-aggregants, surfacing agents, may be added to the granulate prior to coating.
Also provided herein is the use of a granulate composition of *A. galanga* or *A. conchigera* according to the present disclosure optionally together with an extract of *Punica granatum,* for use as a medicament or as a medical device.
Also provided herein is the use of an oily extract of *A. galanga* or *A. conchigera* according to the third aspect optionally together with an extract of *Punica granatum,* for use as a medicament or as a medical device.
Also provided herein is the use of a granulate composition of *A. galanga* or *A. conchigera* according to the second aspect optionally together with an extract of *Punica granatum,* for use in the treatment of male infertility caused by low sperm count and/or by low sperm motility.
Also provided herein is the use of an oily extract of *A. galanga* or *A. conchigera* according to the third aspect optionally together with an extract of *Punica granatum,* for use in the treatment of male infertility caused by low sperm count and/or by low sperm motility.
Also provided herein is the use of a granulate composition of *A. galanga* or *A. conchigera* according to the second aspect optionally together with an extract of *Punica granatum,* for use in the treatment of metabolic syndrome.

Also provided herein is the use of an oily extract of *A. galanga* or *A. conchigera* according to the third aspect optionally together with an extract of *Punica granatum,* for use in the treatment of metabolic syndrome.

Also provided herein is the use of a granulate composition of *A. galanga* or *A. conchigera* according to the second aspect of the present invention, for enhancing fertility in a male subject. The male subject is preferably a mammal, such as, but not limited to, a human or a domestic animal, for example a bull, a sheep, a pig, a horse, a dog or a cat.

Also provided herein is the use of an oily extract of *A. galanga* or *A. conchigera* according to the third aspect of the present invention, for enhancing fertility in a male subject. The male subject is preferably a mammal, such as, but not limited to, a human or a domestic animal, for example a bull, a sheep, a pig, a horse, a dog or a cat.
The granulate composition of *A. galanga* or *A. conchigera* according to the second aspect or the oily extract of *A. galanga* or *A. conchigera* according to the third aspect may be administered together with an extract of mashed fruits of *Punica granatum* (pomegranate) or of *Terminalia catappa* or of *Terminalia myriocarpa* or of *Combretum molle.* Such extract may be obtained by mashing the pericarp of the fruits, e.g. by mashing the remains of squeezed fruits essentially devoid of juice, followed by extraction with eg ethanol. Alternatively, whole fruits may be mashed.

In other instances, the granulate powder of *A. galanga* or *A. conchigera* or the oily extract of *A. galanga* or *A. conchigera* is administered together with a plant extract containing at least 20% of punicalagins.

In one instance, the granulate composition or the oily extract of *A. galanga* or *A. conchigera* and the extract of *Punica granatum* are co-administered to a subject. The subject may suffer from poor semen quality or have normal semen quality. Poor semen quality may be reflected by a low total motile sperm count (TMSC), such as a TMSC<15 x 10⁶. Poor semen quality may also be reflected by low sperm motility. In some embodiments, co-administration of a composition comprising the granulate composition or the oily extract of *A. galanga* or *A. conchigera* and an extract of pomegranate to a male subject results in increased semen quality, for example it results in increased TMSC and/or increased sperm motility and/or increased spermatogenesis. Increased sperm motility may be reflected by an improvement in the motility grade, such as an improvement from grade d to grade c, from grade c to grade b, from grade b to grade a.

Specific instances relate to the use of the granulate composition or the oily extract of *A. galanga* or *A. conchigera* and an extract of *P. granatum* for enhancing male fertility by co-administration to a male subject for a duration of at least 30 days, such as at least 50 days, such as at least 100 days, such as at least 150 days. Preferably, the granulate composition or the oily extract of *A. galanga* or *A. conchigera* and the pomegranate extract are administered at least until the subject has conceived offsprings.

Other instances relate to the use of the granulate composition or the oily extract of *A. galanga* or *A. conchigera* for enhancing male fertility by administration to a male subject of said granulate composition or oily extract at a dosage of at least 100 mg/day, such as at least 125 mg/day, such as at least 250 mg/day, such as between 250 and 3000 mg/day, preferably at least 500 mg/day, such as 225 mg/day. Other embodiments relate to the use of a granulate composition or oily extract of *A. galanga* or *A. conchigera* for enhancing male fertility by administration to a male subject of said granulate composition at a dosage of at least 2 mg ACA/day, such as between 5 and 50 mg ACA/day, such as between 10 and 20 mg ACA/day, preferably at least 10 mg ACA/day.

In some instances, male fertility is enhanced by co-administration of a granulate composition or an oily extract of *A. galanga* or *A. conchigera,* eg. in tablet or liquid dosage form and an extract of *P. granatum* to a male subject. The *P. granatum* extract comprises preferably at least 40% polyphenols, at least 30% punicosides and at least 20% punicalagins. In some embodiments, the granulate composition or the oily extract of *A. galanga* or *A. conchigera* and the extract of *P. granatum* are administered to a male subject at a dosage of at least 75 mg punicalagins/day, such as between 75 mg/day and 600 mg/day, such as between 100 mg/day and 500 mg/day, preferably at least 300 mg/day. In some embodiments, the dosage of punicosides is at least 100 mg/day, such as between 100 mg/day and 800 mg/day, such as between 100 mg/day and 600 mg/day, preferably at least 400 mg/day. The dosage of polyphenols is at least 125 mg/day, such as between 125 mg/day and 1000 mg/day, such as between 123 mg/day and 700 mg/day, preferably at least 500 mg/day.

In some instances, the granulate composition or the oily extract of *A. galanga* or *A. conchigera* and the extract of *P. granatum* are co-formulated into tablets or other suitable, oral unit dosage forms containing the granulate composition or the oily extract of *A. galanga* or *A. conchigera* at a dosage of at least 2 mg ACA/day, such as between 5 and 50 mg ACA/day, preferably at least 10 mg ACA/day, in combination with the extract of *P. granatum,* optionally individually converted into granulate form, at a dosage of at least 75 mg punicalagins/day, such as between 75 mg/day and 600 mg/day, such as between 100 mg/day and 500 mg/day, preferably at least 300 mg/day.

### Examples

### Example 1 Granulation procedure

Longitudinally split, freeze-dried rhizomes were milled on a Co-mill equipped with an air cooling system in order to avoid heating of the rhizomes during the grating process. The tough fibres present in the rhizomes cause significant friction in the grating process. Our initial experiments showed that the temperature in the powder would reach 50°C or more, which is expected to accelerate the hydrolysis of ACA. We therefore applied a standard air cooling system to the mill, which kept the temperature below 30°C. GC quantification of the ACA content showed no significant loss of ACA following milling using the cooling system, and the following sequence of down-sizing on a series of screens: first a 12.7 mm screen, then a 2mm screen and finally a 1 mm screen.

The milled powder needs to be brought into a homogenous mixture with higher density in order to be incorporated into tablets. For this purpose a standard procedure in the industry has been to apply a binder (e.g. polyvinylpyrrolidone) dissolved in a relatively high percentage of water and e.g. organic solvent such as ethanol. We developed a procedure dissolving the binder (7.5% polyvinylpyrrolidone (PVP 90)) rel. to dry material in 99.5% ethanol in order to reduce hydrolysis of ACA.

The grinded, freeze-dried plant material and a PVP-ethanol solution (91.4% w/w ethanol, 8.6% w/w PVP90) were mixed in a mixer at 57 rpm at a temperature not exceeding 27° C. The mixing process lasted for 90 minutes. The ratio between PVP-ethanol solution and grinded plant material was 0.95 (w/w).

Subsequently, the mixture was transferred to a fluid bed dryer by vacuum and the drying process was carried out with process air (1500 m³/hr) under vacuum conditions. During this process, the product temperature did not exceed 30° C. The whole drying procedure lasted for 2 - 3 hours.

The resulting granulated powder was milled on a Co-mill using a 1 mm screen in order to further homogenize the particles prior to formulation into tablets. A standard air cooling system connected to the mill kept the temperature below 30°C. The granulated powder was now suitable for incorporation into tablets.

The distribution of particle sizes following milling is shown in **table 1,** and the specifications of¹⁾ the granulate powder compared with ²⁾freeze-dried, intact rhizomes and ³⁾dry preparation appear from **table 2.**

**Table 1.**

| | Particle size, **µm** | Distribution, percent |
|---|---|---|
| | 2000 - 1000 | 0.10 |
| | 1000 - 500 | 28.90 |
| | 500 - 250 | 35.90 |
| | 250 - 125 | 21.40 |
| | 125 - 63 | 12.00 |
| | 63 - 45 | 1.70 |
| | < 45 | 0,0 |
| **Total** | | **100** |

**Table 2**

| **Preparation** | **Density** (g/100 ml) | **Moisture (%)** | **Angle of repose** |
|---|---|---|---|
| Freeze-dried rhizomes | 9 | 7,75 | |
| Grated material | 13 | 10,39 | N/A (did not run through funnel) |
| Granulated powder | 26 | 8,93 | 35° |

### Example 2 - tabletizing produre

The granulated powder from Example 1 was incorporated into tablets using a standard procedure in the industry. This procedure caused no significant change in the ACA content of the granulated powder (between 7.9 and 8.1%).

Tablets were manufactured, each tablet weighing 457.50 mg, and containing 7.50 mg coating and 120 mg granulate. Table 3 shows an example of tablets comprising a granulate of *A. galanga* and table 4 shows the results of the microbiological tests performed before granulation and on the final tablets.

**Table 3.**

| mg/ tablet | Compound | Function |
|---|---|---|
| 120 | Granulate of *Alpinia galanga* prepared according to the procedures described in example 1. | Active ingredient |
| 200 | Microcrystalline cellulose (E460) | Bulking agent |
| 100 | Dicalcium phosphate (E341) | Bulking agent |
| 11 | Crosslinked sodium carboxy methyl cellulose (E468) | Bulking agent |
| 11 | Sodium carboxy methyl cellulose (E466) | Bulking agent |
| 5 | Silicon dioxide (E551) | Anti-caking agent |
| 3 | Magnesium salts of veg. fatty acids (E470b) | Glazing agent |
| 7,5 | Vivacoat^{®} - hydroxypropyl methyl cellulose (E464) / titanium dioxide (E171) / polyethylene glycol (1521) | Filmcoat |
| 61 | Purified water | Carrier for filmcoat |
| 450 | Total core tablet | |
| 457,5 | Total tablet with film coat | |

### Specifications for tablets:

Unit size: 9,5 x 9,5 x 5,25 mm (without coating)
Height after coating: 5,35 mm
Hardness: 125 N before coating - 150 N after coating
Disintegration time: less than 15 minutes

**Table 4 Microbiological test**

| | **Results** | |
|---|---|---|
| **Parameter measured** | **Before granulation** | **Tabletted granulate** |
| Enterobacteriaceae 37°C | 160 CFU/g | < 10 CFU/g |
| Aerobe germs 30°C / 3d | 12000 CFU/g | 170 CFU/g |
| Mould | < 10 CFU/g | < 10 CFU/g |
| Yeast | 2500 CFU/g | < 10 CFU/g |

### References

Agbaje, I. M., Rogers, D. A., McVicar, C. M., McClure, N., Atkinson, A. B., Mallidis, C., & Lewis, S. E. (2007). Insulin dependant diabetes mellitus: implications for male reproductive function. Hum Reprod, 22(7), 1871-1877. doi: 10.1093/humrep/dem077
Akhtar, M. S., Khan, M. A., & Malik, M. T. (2002). Hypoglycaemic activity of Alpinia galanga rhizome and its extracts in rabbits. Fitoterapia, 73(7-8), 623-628.
Islam, M. W., Zakaria, M. N. M., Radhakrishnan, R., Liu, X. M., Ismail, A., Chan, K., & Al-Attas, A. (2000). Galangal (Alpinia galanga Willed.) and black seeds (Nigella sativa Linn.) and sexual stimulation in male mice. Journal of Pharmacy & Pharmacology, 52(Supplement), 278.
lyer, D., Sharma, B. K., & Patil, U. (2013). Isolation of Bioactive Phytoconstituent from Alpinia galanga L. with Anti-Hyperlipidemic Activity. Journal of Dietary Supplements, 10(4), 309-317.
Kaspersen, M., H. Jakobsen, I. Giversen, L. Christensen and J. Fedder (2013). "Punalpin (R) Increases the Numbers of Motile Sperm in Men with Reduced Semen Quality: A Prospective, Randomized, Controlled, Double-Blinded Trial." Human Reproduction 28: 10-11.
Kumar, S., & Alagawadi, K. (2011). Influence of Alpinia galanga rhizomes on cafeteria diet induced obesity in rats. Journal of Natural Remedies, 11(2), 158-166.
Kumar, S., & Alagawadi, K. (2013). Anti-obesity effects of galangin, a pancreatic lipase inhibitor in cafeteria diet fed female rats. Pharmaceutical Biology, 51(5), 607-613.
Leisegang, K., Udodong, A., Bouic, P. J., & Henkel, R. R. (2014). Effect of the metabolic syndrome on male reproductive function: a case-controlled pilot study. Andrologia, 46(2), 167-176. doi: 10.1111/and.12060
Nakamura, Y., Murakami, A., Ohto, Y., Torikai, K., Tanaka, T., & Ohigashi, H. (1998). Suppression of tumor promoter-induced oxidative stress and inflammatory responses in mouse skin by a superoxide generation inhibitor 1'-acetoxychavicol acetate. Cancer Research, 58(21), 4832-4839.
Ohnishi R, Matsui-Yuasa I, Deguchi Y, Yaku K, Tabuchi M, Munakata H, Akahoshi Y, Kojima-Yuasa A. (2012) 1'-acetoxychavicol acetate inhibits adipogenesis in 3T3-L1 adipocytes and in high fat-fed rats. Am J Chin Med. 40(6):1189-204.
Qureshi, S., Shah, A. H., & Ageel, A. M. (1992). Toxicity Studies on Alpinia galanga and Curcuma longa. Planta Medica, 58(2), 124-127.
Seo JW, Cho SC, Park SJ, Lee EJ, Lee JH, Han SS, Pyo BS, Park DH, Kim BH (2013). 1'-Acetoxychavicol acetate isolated from Alpinia galanga ameliorates ovalbumin-induced asthma in mice. PLoS One. 2013;8(2):e56447.
Sivakumar, A. S., Viswanathan, P., & Anuradha, C. V. (2010). Dose-dependent effect of galangin on fructose-mediated insulin resistance and oxidative events in rat kidney. Redox Report, 15(5), 224-232.
Srividya, A. R., Dhanabal, S. P., Satish Kumar, M. N., & Bavadia, P. K. H. (2011). Antioxidant and antidiabetic activity of Alpinia galanga. International Journal of Pharmacognosy and Phytochemical Research, 3(1), 6-12.
Tremellen, K. (2008). Oxidative stress and male infertility - a clinical perspective. Human Reproduction Update, 14(3), 243-258.
Yang, X.G. and Eilerman, R.G. (1999) Pungent principal of Alpinia galangal (L.) Swartz and its applications. Journal of Agricultural and Food Chemistry 47, 4, 1657-1662.
Ye Y, Li B. (2006) 1'S-1'-acetoxychavicol acetate isolated from Alpinia galanga inhibits human immunodeficiency virus type 1 replication by blocking Rev transport. J Gen Virol. 87:2047-53

## Claims

1. A method for preparing a granulate composition of the rhizomes of *Alpinia galanga* or *Alpinia conchigera,* said method comprising the steps of:
a. providing a dry preparation of the rhizomes of *Alpinia galanga* or *Alpinia conchigera* which is milled;
b. suspending said dry preparation in an essentially pure organic solvent,
c. wet granulation of said dry preparation with a solution consisting of a binder dissolved in an essentially pure organic solvent, said solution being essentially devoid of water;
d. removing the organic solvent;
e. providing a final milling;
wherein steps b-d) are performed at a temperature lower than 50°C, such as lower than 40°C, such as lower than 35°C, such as 30°C, and wherein said granulate composition comprises at least 2% 1'S-1'-acetoxychavicol acetate.

2. The method of claim 1, in which the organic solvent is ethanol.

3. The method of any one of claims 1 to 2, in which step d) is performed under vacuum.

4. The method of any one of claims 1 to 3, wherein steps b) and c) are combined, such that the dry preparation of the rhizomes of *Alpinia galanga* or *Alpinia conchigera* is directly mixed with a solution comprising a binder instead of first being suspended in an essentially pure organic solvent.

5. The method of any one of claims 1 to 4, in which step a) comprises a downsizing step.

6. The method according to claims 1 to 5, wherein all the steps a) - e) are performed at a temperature of 30°C or less.

7. A granulate composition of rhizomes from *Alpinia galanga* or *Alpinia conchigera,* said granulate composition comprising:
i) all the constituent parts of *Alpinia galanga* or *Alpinia conchigera* in essentially anhydrous, or desiccated form;
ii) at least 2% 1'S-1'-acetoxychavicol acetate.

8. The granulate composition according to claim 7, comprising at least 2.5% 1'S-1'-acetoxychavicol acetate, such as at least 3%, such as at least 3.5%, such as at least 4%, such as at least 4.5%, such as at least 5%, such as at least 5.5%, such as at least 6%, such as at least 6.5%, such as at least 7%, such as at least 7.5%, such as at least 8%.

9. The granulate composition according to claim 7, obtainable by the method according to any one of claims 1 to 7.

10. The granulate composition according to any one of claims 7 to 9, further comprising one or more of the following compounds: 1'S-1'-acetoxyeugenol acetate, 1'S-1'-hydroxychavicol acetate, p-hydroxycinnamaldehyde, p-coumaryl-diacetate, trans-coniferyl-diacetate, trans-p-coumaryl alcohol, trans-p-hydroxycinnamyl acetate, p-acetoxycinnamyl alcohol, p-hydroxybenzaldehyde, chavicol acetate, chavicol, methyl-eugenol, eugenol, eugenol acetate, methyl cinnamate; terpenes and related compounds, including monoterpenes and sesquiterpenes, such as 1,8-cineole, α-pinene, β-pinene, α-terpineol, terpinen-4-ol or 4-terpineol, camphene, camphor, myrcene, (Z)-β-ocimene, limonene, linalool, fenchyl acetate, geranyl acetate, bornyl acetate, citronellyl acetate, 2-acetoxy-1,8-cineole, 3-acetoxy-1,8-cineole, guaiol, β-farnesene, β-bisabolene, (Z,E)-farnesol, β-caryo¬phyllene or α-bergamotene.

11. The granulate composition according to any one of claims 7 to 10 formulated with pharmaceutically acceptable, commonly used excipients or coatings.

12. The granulate composition according to any one of claims 7 to 11, formulated as an ingestible preparation, such as a tablet, a pill, a capsule, a powder or a suspension in an edible oil such as flaxseed oil, olive oil, sunflower oil, corn oil, peanut oil and grape seed oil.

13. The granulate composition according to any one of claims 7 to 11, formulated as a dietary supplement, a food additive or as a medical food.

14. The granulate composition according to any one of claims 7 to 11, optionally together with an extract of *Punica granatum,* for use as a medicament.

## Patentansprüche

1. Verfahren zur Herstellung einer Granulatzusammensetzung der Rhizome von *Alpinia galanga* oder *Alpinia conchigera,* wobei das Verfahren die folgenden Schritte umfasst:
a. Bereitstellen eines Trockenpräparats der Rhizome von *Alpinia galanga* oder *Alpinia conchigera,* das vermahlen wird;
b. Suspendieren des Trockenpräparats in einem im Wesentlichen reinen organischen Lösungsmittel,
c. Nassgranulierung des Trockenpräparats mit einer Lösung, die aus einem Bindemittel, das in einem im Wesentlichen reinen organischen Lösungsmittel gelöst ist, besteht, wobei die Lösung im Wesentlichen wasserfrei ist;
d. Entfernen des organischen Lösungsmittels;
e. Durchführen eines abschließenden Mahlvorgangs;
wobei die Schritte b-d) bei einer Temperatur von niedriger als 50°C, wie niedriger als 40°C, wie niedriger als 35°C, wie 30°C, durchgeführt werden und wobei die Granulatzusammensetzung mindestens 2% 1'S-1'-Acetoxychavicolacetat umfasst.

2. Verfahren nach Anspruch 1, wobei es sich bei dem organischen Lösungsmittel um Ethanol handelt.

3. Verfahren nach einem der Ansprüche 1 bis 2, wobei Schritt d) im Vakuum durchgeführt wird.

4. Verfahren nach einem der Ansprüche 1 bis 3, wobei die Schritte b) und c) so kombiniert werden, dass das Trockenpräparat der Rhizome von *Alpinia galanga* oder *Alpinia conchigera* direkt mit einer Lösung, die ein Bindemittel umfasst, vermischt wird, statt erst in einem im Wesentlichen reinen organischen Lösungsmittel suspendiert zu werden.

5. Verfahren nach einem der Ansprüche 1 bis 4, wobei Schritt a) einen Größenverminderungsschritt umfasst.

6. Verfahren nach den Ansprüchen 1 bis 5, wobei alle Schritte a)-e) bei einer Temperatur von 30°C oder weniger durchgeführt werden.

7. Granulatzusammensetzung von Rhizomen von *Alpinia galanga* oder *Alpinia conchigera,* wobei die Granulatzusammensetzung Folgendes umfasst:
i) alle Bestandteile von *Alpinia galanga* oder *Alpinia conchigera* in im Wesentlichen wasserfreier oder getrockneter Form;
ii) mindestens 2% 1'S-1'-Acetoxychavicolacetat.

8. Granulatzusammensetzung nach Anspruch 7, umfassend mindestens 2,5% 1'S-1'-Acetoxychavicolacetat, wie mindestens 3%, wie mindestens 3,5%, wie mindestens 4%, wie mindestens 4,5%, wie mindestens 5%, wie mindestens 5,5%, wie mindestens 6%, wie mindestens 6,5%, wie mindestens 7%, wie mindestens 7,5%, wie mindestens 8%.

9. Granulatzusammensetzung nach Anspruch 7, die nach dem Verfahren nach einem der Ansprüche 1 bis 7 erhältlich ist.

10. Granulatzusammensetzung nach einem der Ansprüche 7 bis 9, die weiterhin eine oder mehrere der folgenden Verbindungen umfasst: 1'S-1'-Acetoxyeugenolacetat, 1'S-1'-Hydroxychavicolacetat, p-Hydroxycinnamaldehyd, p-Cumaryldiacetat, trans-Coniferyldiacetat, trans-p-Cumarylalkohol, trans-p-Hydroxycinnamylacetat, p-Acetoxycinnamylalkohol, p-Hydroxybenzaldehyd, Chavicolacetat, Chavicol, Methyleugenol, Eugenol, Eugenolacetat, Methylcinnamat; Terpene und verwandte Verbindungen, einschließlich Monoterpene und Sesquiterpene, wie 1,8-Cineol, α-Pinen, β-Pinen, α-Terpineol, Terpinen-4-ol oder 4-Terpineol, Camphen, Campher, Myrcen, (Z)-β-Ocimen, Limonen, Linalool, Fenchylacetat, Geranylacetat, Bornylacetat, Citronellylacetat, 2-Acetoxy-1,8-cineol, 3-Acetoxy-1,8-cineol, Guaiol, β-Farnesen, β-Bisabolen, (Z,E)-Farnesol, β-Caryophyllen oder α-Bergamoten.

11. Granulatzusammensetzung nach einem der Ansprüche 7 bis 10, die mit pharmazeutisch unbedenklichen, üblicherweise verwendeten Trägerstoffen oder Beschichtungen formuliert ist.

12. Granulatzusammensetzung nach einem der Ansprüche 7 bis 11, die als Präparat zum Einnehmen, wie als Tablette, als Pille, als Kapsel, als Pulver oder als Suspension in einem Speiseöl, wie Leinöl, Olivenöl, Sonnenblumenöl, Maiskeimöl, Erdnussöl und Traubenkernöl, formuliert ist.

13. Granulatzusammensetzung nach einem der Ansprüche 7 bis 11, die als Nahrungsergänzungsmittel, als Nahrungszusatzstoff oder als medizinische Nahrung formuliert ist.

14. Granulatzusammensetzung nach einem der Ansprüche 7 bis 11, die gegebenenfalls gemeinsam mit einem Extrakt von *Punica granatum* formuliert ist, zur Verwendung als Arzneimittel.

## Revendications

1. Méthode de préparation d'une composition granulaire des rhizomes d'*Alpinia galanga* ou d'*Alpinia conchigera,* ladite méthode comprenant les étapes :
a. de fourniture d'une préparation sèche des rhizomes d'*Alpinia galanga* ou d'*Alpinia conchigera,* qui est broyée ;
b. de mise en suspension de ladite préparation sèche dans un solvant organique essentiellement pur ;
c. de granulation par voie humide de ladite préparation sèche avec une solution constituée d'un liant solubilisé dans un solvant organique essentiellement pur, ladite solution étant essentiellement dépourvue d'eau ;
d. d'élimination du solvant organique ;
e. de fourniture d'un broyage final ;
où les étapes b-d) sont effectuées à une température inférieure à 50°C, telle qu'inférieure à 40°C, telle qu'inférieure à 35°C, telle que de 30°C, et où ladite composition granulaire comprend au moins 2% d'acétate de 1'S-1'-acétoxychavicol.

2. Méthode selon la revendication 1, dans laquelle le solvant organique est l'éthanol.

3. Méthode selon l'une quelconque des revendications 1 à 2, dans laquelle l'étape d) est effectuée sous vide.

4. Méthode selon l'une quelconque des revendications 1 à 3, dans laquelle les étapes b) et c) sont combinées, de sorte que la préparation sèche des rhizomes d'*Alpinia galanga* ou d'*Alpinia conchigera* est mélangée directement avec une solution comprenant un liant, au lieu d'être d'abord mise en suspension dans un solvant organique essentiellement pur.

5. Méthode selon l'une quelconque des revendications 1 à 4, dans laquelle l'étape a) comprend une étape de rapetissement.

6. Méthode selon les revendications 1 à 5, dans laquelle les étapes a)-e) sont effectuées à une température de 30°C ou moins.

7. Composition granulaire de rhizomes d'*Alpinia galanga* ou d'*Alpinia conchigera,* ladite composition granulaire comprenant :
i) toutes les parties constituantes d'*Alpinia galanga* ou d'*Alpinia conchigera* sous forme essentiellement anhydre, ou desséchée ;
ii) au moins 2% d'acétate de 1'S-1'-acétoxychavicol.

8. Composition granulaire selon la revendication 7, comprenant au moins 2,5% d'acétate de 1'S-1'-acétoxychavicol, tel qu'au moins 3%, tel qu'au moins 3,5%, tel qu'au moins 4%, tel qu'au moins 4,5%, tel qu'au moins 5%, tel qu'au moins 5,5%, tel qu'au moins 6%, tel qu'au moins 6,5%, tel qu'au moins 7%, tel qu'au moins 7,5%, tel qu'au moins 8%.

9. Composition granulaire selon la revendication 7, pouvant être obtenue par la méthode selon l'une quelconque des revendications 1 à 7.

10. Composition granulaire selon l'une quelconque des revendications 7 à 9, comprenant en outre l'un ou plusieurs parmi les composés suivants : l'acétate de 1'S-1'-acétoxyeugénol, l'acétate de 1'S-1'-hydroxychavicol, le p-hydroxycinnamaldéhyde, le diacétate de p-coumaryle, le diacétate de trans-coniféryle, l'alcool trans-p-coumarylique, l'acétate de trans-p-hydroxycinnamyle, l'alcool p-acétoxycinnamylique, le p-hydroxybenzaldéhyde, l'acétate de chavicol, le chavicol, le méthyl-eugénol, l'eugénol, l'acétate d'eugénol, le cinnamate de méthyle ; les terpènes et les composés apparentés, y compris les monoterpènes et les sesquiterpènes, tels que le 1,8-cinéol, l'α-pinène, le β-pinène, l'α-terpinéol, le terpinène-4-ol ou le 4-terpinéol, le camphène, le camphre, le myrcène, le (Z)-β-ocimène, le limonène, le linalool, l'acétate de fenchyle, l'acétate de géranyle, l'acétate de bornyle, l'acétate de citronellyle, le 2-acétoxy-1,8-cinéol, le 3-acétoxy-1,8-cinéol, le guaïol, le β-farnesène, le β-bisabolène, le (Z,E)-farnésol, le β-caryophyllène ou l'α-bergamotène.

11. Composition granulaire selon l'une quelconque des revendications 7 à 10, formulée avec des excipients ou des revêtements pharmaceutiquement acceptables et couramment utilisés.

12. Composition granulaire selon l'une quelconque des revendications 7 à 11, formulée comme préparation ingérable, telle qu'un comprimé, une pilule, une capsule, une poudre ou une suspension dans une huile comestible telle qu'une huile de graines de lin, une huile d'olive, une huile de tournesol, une huile de maïs, une huile d'arachide et une huile de pépins de raisin.

13. Composition granulaire selon l'une quelconque des revendications 7 à 11, formulée comme complément alimentaire, additif alimentaire ou comme aliment médicamenteux.

14. Composition granulaire selon l'une quelconque des revendications 7 à 11, éventuellement conjointement avec un extrait de *Punica granatum,* pour une utilisation comme médicament.
